# EUROPEAN PATENT APPLICATION

(11) **EP 4 488 309 A1**
(43) Date of publication of application: **08.01.2025**
(21) Application number: 23763519.8
(22) Date of filing: 01.03.2023
(51) Int. Cl.: C08G 18/38, C08G 18/77, G02B 1/04

(54) **RESIN, MOLDED ARTICLE, OPTICAL MATERIAL, LENS, 2,5-BIS(ISOCYANATOMETHYL)FURAN, AND POLYMERIZABLE COMPOSITION**

(30) Priority: 01.03.2022 JP 2022031204
(71) Applicant: Mitsui Chemicals, Inc., Tokyo 104-0028 (JP)
(72) Inventor: HANAWA, Takayuki, Omuta-shi, Fukuoka 836-8610 (JP)
(74) Representative: Mewburn Ellis LLP
(86) International application number: PCT/JP2023/007696
(87) International publication number: WO 2023/167262

(57) **Abstract**

A resin includes a cured product of a polymerizable composition including a compound represented by the following general formula (1) and a thiol compound having one or more mercapto groups, and a biomass degree of the resin is 15% or more. In the general formula (1), each of R₁ and R₂ independently represent a hydrogen atom or a methyl group, and n represents an integer of 0 or 1.

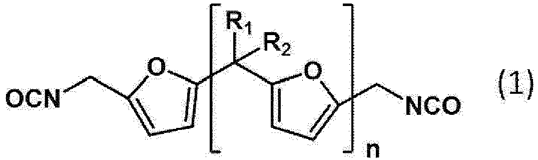

## Description

### Technical Field

The present disclosure relates to a resin, a molded body, an optical material, a lens, 2,5-bis(isocyanatomethyl)furan, and a polymerizable composition.

Plastic lenses, which are lenses including a resin, are lighter and less likely to break than inorganic lenses, and can be dyed, so in recent years they have rapidly become popular for use in eyeglass lenses, camera lenses, and other applications.

For example, lenses including a thiourethane resin have been studied. Patent Document 1 proposes a polymerizable composition for optical materials that including a polyisocyanate (a) including an aliphatic polyisocyanate (a1) and a modified product (a2) of an aliphatic polyisocyanate, and a bifunctional or higher functional polythiol (b) having mercapto groups, and the modified product (a2) is included in an amount of 60% by weight or less in the polyisocyanate (a), as well as a molded body and a plastic eyeglass lens that use a thiourethane resin obtained by polymerizing and curing the composition.

### [Patent Document 1] International Publication No. 2015/119220

### SUMMARY OF INVENTION

### Technical Problem

Patent Document 1 discloses a configuration in which the biomass degree of a thiourethane resin obtained by polymerizing and curing a polymerizable composition for optical materials is 25% or more. Considering the impact on the global environment, it is desirable to obtain a resin using a biomass raw material other than the thiourethane resin disclosed in Patent Document 1. In addition, in a thiourethane resin with the refractive index of more than 1.65, there is room for improvement in terms of achieving both a refractive index and a high biomass degree, in terms of productivity, or the like.

The object of the present disclosure is to provide a resin obtained by using a compound derived from a biomass raw material, and a molded body, an optical material, and a lens each of which includes the resin, and 2,5-bis(isocyanatomethyl)furan derived from a biomass raw material and a polymerizable composition including thereof.

### Solution to Problem

The specific means to solve the above problems include the following aspects.
<1> A resin, comprising a cured product of a polymerizable composition including a compound represented by the following general formula (1) and a thiol compound having one or more mercapto groups,
   wherein a biomass degree of the resin is 15% or more. 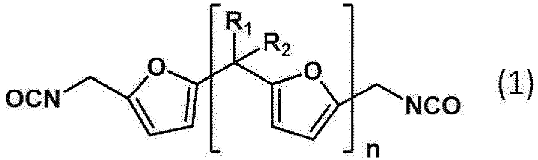 In the general formula (1), each of R₁ and R₂ independently represent a hydrogen atom or a methyl group. n represents an integer of 0 or 1.
<2> The resin according to <1>, wherein the compound represented by the general formula (1) includes 2,5-bis(isocyanatomethyl)furan represented by the following formula (2). 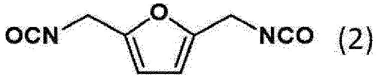
<3> The resin according to <1> or <2>, wherein the biomass degree of the resin is 35% or more.
<4> The resin according to any one of <1> to <3>, wherein the thiol compound includes at least one compound selected from the group consisting of 4-mercaptomethyl-1,8-dimercapto-3,6-dithiaoctane,
   4,8-dimercaptomethyl-1,11-dimercapto-3,6,9-trithiaundecane,
   4,7-dimercaptomethyl-1,11-dimercapto-3,6,9-trithiaundecane, and
   5,7-dimercaptomethyl-1,11-dimercapto-3,6,9-trithiaundecane.
<5> The resin according to any one of <1> to <4>, wherein the polymerizable composition further includes an episulfide compound having one or more episulfide groups.
<6> The resin according to any one of <1> to <5>, wherein a refractive index ne of the resin is 1.650 or more.
<7> A molded body, comprising the resin according to any one of <1> to <6>.
<8> An optical material, comprising the molded body according to <7>.
<9> A lens, comprising the molded body according to <7>.
<10> 2,5-bis(isocyanatomethyl)furan, having a biomass degree of 50% or more.
<11> 2,5-bis(isocyanatomethyl)furan, derived from 2,5-bis(aminomethyl)furan obtained from a biomass raw material.
<12> A polymerizable composition, comprising the 2,5-bis(isocyanatomethyl)furan according to <10> or <11>.
<13> The polymerizable composition according to <12>, further comprising a thiol compound having one or more mercapto groups.
<14> The polymerizable composition according to <12> or <13>, further comprising an episulfide compound having one or more episulfide groups.

### Advantageous Effects of Invention

According to the present disclosure, it is possible to provide a resin obtained by using a compound derived from a biomass raw material, and a molded body, an optical material, and a lens each of which includes the resin, and 2,5-bis(isocyanatomethyl)furan derived from a biomass raw material and a polymerizable composition including thereof.

### DESCRIPTION OF EMBODIMENTS

In the present disclosure, those numerical ranges that are expressed with "to" each denote a range that includes the numerical values stated before and after "to" as the minimum value and the maximum value, respectively.

In the present disclosure, when there are plural kinds of substances that correspond to a component in a composition, the indicated content of the component means, unless otherwise specified, the total content of the plural kinds of substances included in the composition.

In a set of numerical ranges that are stated stepwise in the present disclosure, the upper limit value or the lower limit value of a numerical range may be replaced with the upper limit value or the lower limit value of other numerical range. Further, in a numerical range stated in the present disclosure the upper limit value or the lower limit value of the numerical range may be replaced with a value indicated in Examples.

### <Resin>

A resin of the present disclosure includes a cured product of a polymerizable composition including a compound represented by the following general formula (1) and a thiol compound (hereinafter, also referred to as "thiol compound") having one or more mercapto groups, and the biomass degree of the resin is 15% or more.

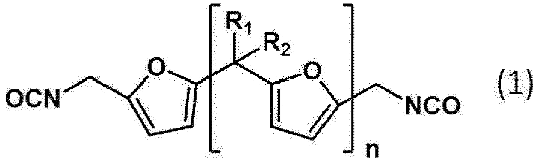

In the general formula (1), each of R₁ and R₂ independently represent a hydrogen atom or a methyl group, and n represents an integer of 0 or 1.

The resin of the present disclosure is a cured product of a polymerizable composition including a compound represented by the general formula (1) and a thiol compound, and has the biomass degree of 15% or more. Therefore, at least one of the compound represented by the general formula (1) and the thiol compound is derived from a biomass raw material. By using a resin obtained from a compound derived from a biomass raw material, it is possible to contribute to the preservation of the global environment and to produce molded bodies, optical materials, lenses, or the like that are in harmony with the global environment. The cured product of the polymerizable composition including the compound represented by the general formula (1) and the thiol compound tends to have a higher refractive index than a cured product of a polyurethane resin.

In the general formula (1), R₁ and R₂ may be a hydrogen atom or a methyl group. The compound represented by the general formula (1) may be only one type of compound, or may be a mixture of two or more types of compounds.

From the viewpoint of operability during distillation purification in compound production and the heat resistance of the resulting resin, the compound represented by the general formula (1) is a 2,5-bis(isocyanatomethyl)furan represented by the following formula (2) is preferred. 2,5-bis(isocyanatomethyl)furan corresponds to a compound where n=0 in the general formula (1). Furthermore, by using 2,5-bis(isocyanatomethyl)furan represented by the following formula (2), the refractive index of the resulting resin tends to be further increased.

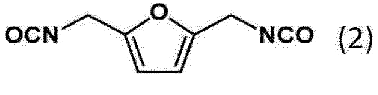

The biomass degree of the compound represented by the general formula (1), preferably the biomass degree of 2,5-bis(isocyanatomethyl)furan, is preferably 50% or more, more preferably 60% or more. From the viewpoint of availability, the biomass degree of the compound represented by the general formula (1), preferably the biomass degree of 2,5-bis(isocyanatomethyl)furan, may be 80% or less.

In this disclosure, the biomass degree (%) is the biomass degree of the sample calculated using δ¹³C-corrected pMC according to ASTM D6866-21. The atmospheric correction coefficient REF (pMC) was calculated using REF (pMC) = 100, assuming that the biomass material was produced from 2019 to 2021.

### Test method

Using a ¹⁴C-AMS dedicated device (manufactured by NEC Corporation) based on tandem accelerator, measurements of ¹⁴C counts, ¹³C concentration (¹³C/¹²C) and¹⁴C concentration (¹⁴C/¹²C) were performed. In the measurement, oxalic acid (HOxII) provided by the National Institute of Standards and Technology (NIST) was used as a standard sample.

δ¹³C is the value represented by measuring ¹³C concentration (¹³C/¹²C) of the sample carbon and expressed in 1,000 minutes deviation (‰) from the reference sample.

pMC (percent Modern Carbon) is the ratio of ¹⁴C concentration of the sample carbon to standard modern carbon.

δ¹⁴C is the value that the deviation of the ¹⁴C concentration of the sample carbon to standard modern carbon is expressed by 1,000 minutes deviation (‰) and Δ¹⁴C is the value obtained by correcting this with δ¹³C.

Hereinafter, an example of a manufacturing method of the compound represented by the general formula (1) will be explained. Note that the manufacturing method of the compound represented by the general formula (1) may be any method as long as it can manufacture the compound represented by the general formula (1) using a biomass raw material, and is not limited to the manufacturing method described below.

### (Manufacturing method 1 of compound represented by general formula (1))

First, an example of a method of manufacturing 2,5-bis(isocyanatomethyl)furan, which is a type of compound represented by the general formula (1), using a biomass raw material will be explained. A saccharide is prepared as a biomass raw material. The saccharide is not particularly limited, and example thereof include a monosaccharide such as glucose or fructose, a disaccharide such as sucrose or maltose, and a polysaccharide such as cellulose or starch.

Using the prepared saccharide, 5-hydroxymethylfurfural, which is a compound represented by the following formula (3), is obtained through a dehydration reaction or the like. For example, a polysaccharide such as cellulose or starch, and a disaccharide such as sucrose or maltose may be decomposed into glucose by the action of an enzyme, and 5-hydroxymethylfurfural may be obtained by a dehydration reaction of the decomposed glucose, or the like. When synthesizing 5-hydroxymethylfurfural from glucose, fructose may be an intermediate.

Other methods of synthesizing 5-hydroxymethylfurfural from a saccharide include, for example, a method of synthesizing 5-hydroxymethylfurfural from a saccharide such as cellulose, an oligosaccharide, and a monosaccharide, as described in Japanese Patent No. 6328990.

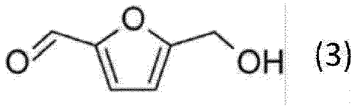

An aldehyde group in 5-hydroxymethylfurfural represented by the formula (3) may be reduced using a reducing agent or the like to form 2,5-bis(hydroxymethyl)furan, which is a compound represented by the formula (5). By reacting ammonia with 2,5-bis(hydroxymethyl)furan in the presence of a catalyst, 2,5-bis(aminomethyl)furan, which is a compound represented by the formula (4), may be obtained as shown in the following reaction formula.

As a method of synthesizing the compound represented by the formula (4) from the compound represented by the formula (5), the synthesis method described in "Chemical Science, 2020, 11, 9884-9890" may be used.

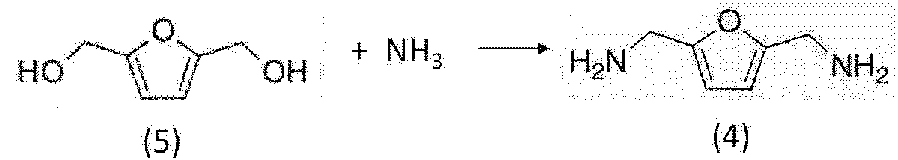

Next, the amino group in 2,5-bis(aminomethyl)furan is phosgenated to obtain 2,5-bis(isocyanatomethyl)furan. A method of obtaining 2,5-bis(isocyanatomethyl)furan includes reaction of at least one of 2,5-bis(aminomethyl)furan or the hydrochloride of 2,5-bis(aminomethyl)furan with phosgene. For the reaction in which an amino group or amine hydrochloride is reacted with phosgene to convert it into an isocyanate group, known manufacuturing methods for isocyanate compounds can be appropriately referred to.

The reaction temperature when phosgenating the amine hydrochloride is preferably 170°C or lower, more preferably 150°C or lower, from the viewpoint of suppressing a side reaction due to dissociation of the amine hydrochloride.

### (Manufacturing method 2 of compound represented by general formula (1))

First, another example of a method of manufacturing a compound represented by the general formula (6), which is a type of compound represented by the general formula (1), using a biomass raw material will be explained.

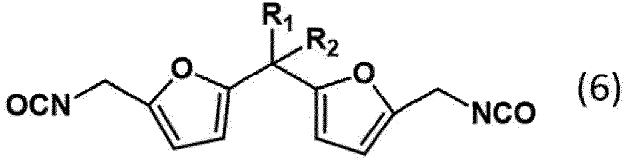

It is preferable to prepare furfural derived from a biomass raw material as a raw material for the compound represented by the general formula (6). For example, a saccharide such as xylose or hemicellulose is prepared as a biomass raw material. Hemicellulose may be decomposed into xylose by the action of an enzyme. Furfural is obtained through the dehydration reaction of xylose. Thereafter, as shown in the following reaction formula, furfurylamine is obtained from furfural by a conventionally known method using a catalyst. After converting furfurylamine into the hydrochloride, the hydrochloride is reacted with a carbonyl compound such as acetone and phosgenation of an amino group derived from furfurylamine is performed by a conventionally known method to obtain the compound represented by the general formula (6).

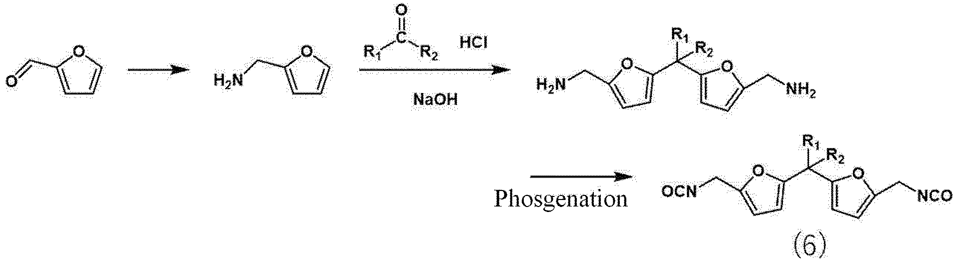

The polymerizable composition includes a thiol compound having one or more mercapto groups together with the compound represented by the general formula (1).

Examples of the thiol compound include a polythiol compound having two or more mercapto groups, a hydroxythiol compound having one or more mercapto groups and one or more hydroxy groups, and the like. Among these, a polythiol compound having two or more mercapto groups and a hydroxythiol compound having one or more mercapto groups and one or more hydroxy groups are preferred.

As the thiol compound, an oligomer of the thiol compound having one or more mercapto groups, a halogen-substituted product (for example, chlorine-substituted product, bromine-substituted product, or the like) of the above-mentioned thiol compound having one or more mercapto groups, or the like may be used.

Further, the thiol compound may be used alone or in combination of two or more types.
The thiol compound may or may not be derived from a biomass raw material.

### (Polythiol compound having two or more mercapto groups)

Examples of a polythiol compound having two or more mercapto groups (hereinafter also referred to as "polythiol compound") include compounds exemplified in International Publication No. 2016/125736.

Examples of the polythiol compound include 4-mercaptomethyl-1,8-dimercapto-3,6-dithiaoctane, 4,8-dimercaptomethyl-1,11-dimercapto-3,6,9-trithiaundecane, 4,7-dimercaptomethyl-1,11-dimercapto-3,6,9-trithiaundecane, 5,7-dimercaptomethyl-1,11-dimercapto-3,6,9-trithiaundecane, pentaerythritol tetrakis(3- mercaptopropionate), bis(mercaptoethyl)sulfide, pentaerythritol tetrakis(2-mercaptoacetate), 2,5-bis(mercaptomethyl)-1,4-dithiane, 1,1,3,3-tetrakis(mercaptomethylthio)propane, 4,6-bis(mercaptomethylthio)-1,3-dithiane, 2-(2,2-bis(mercaptomethylthio)ethyl)-1,3-dithiethane and the like.

The polythiol compound preferably includes at least one compound selected from the group consisting of 4-mercaptomethyl-1,8-dimercapto-3,6-dithiaoctane, 4,8-dimercaptomethyl-1,11-dimercapto-3,6,9-trithiaundecane, 4,7-dimercaptomethyl-1,11-dimercapto-3,6,9-trithiaundecane, and 5,7-dimercaptomethyl-1,11-dimercapto-3,6,9-trithiaundecane.

(Hydroxythiol compound having one or more mercapto groups and one or more hydroxy groups)

Examples of the hydroxythiol compound having one or more mercapto groups and one or more hydroxy groups include 2-mercaptoethanol, 3-mercapto-1,2-propanediol, glycerin bis(mercaptoacetate), 4-mercaptophenol, 2,3-dimercapto-1-propanol, pentaerythritol tris (3-mercaptopropionate), pentaerythritol tris(thioglycolate), and the like.

The polymerizable composition may further include a composition including another active hydrogen compound other than the compound having one or more mercapto groups. Examples of another active hydrogen compound include a polyol compound having two or more hydroxy groups and an amine compound.

### (Polyol compound having two or more hydroxy groups)

The polyol compound having two or more hydroxy groups includes one or more aliphatic or alicyclic alcohols. Specifically, Examples thereof include a linear or branched aliphatic alcohol, an alicyclic alcohol, and an alcohol obtained by adding at least one selected from the group consisting of ethylene oxide, propylene oxide, and ε-caprolactone to the alcohol and the like. More specifically, compounds exemplified in International Publication No. 2016/125736 can be mentioned.

Examples of the polyol compound having two or more hydroxy groups include ethylene glycol, diethylene glycol, triethylene glycol, propylene glycol, 1,3-propanediol, 1,2-cyclopentanediol, 1,3-cyclopentanediol, 1,2-cyclohexanediol, 1,3-cyclohexanediol, 1,4-cyclohexanediol, and the like.

### (Amine compound)

Examples of the amine compound include primary polyamine compounds such as ethylenediamine, 1,2- or 1,3-diaminopropane, 1,2-, 1,3- or 1,4-diaminobutane, 1,5-diaminopentane, 1,6-diaminohexane, 1,7-diaminoheptane, 1,8-diaminooctane, 1,10-diaminodecane, 1,2-, 1,3- or 1,4-diaminocyclohexane, o-, m- or p-diaminobenzene, 3,4- or 4,4'-diaminobenzophenone, 3,4- or 4,4'-diaminodiphenyl ether, 4,4'-diaminodiphenylmethane, 4,4'-diaminodiphenyl sulfide, 3,3' or 4,4 '-diaminodiphenyl sulfone, 2,7-diaminofluorene, 1,5-, 1,8- or 2,3-diaminonaphthalene, 2,3-, 2,6- or 3,4-diaminopyridine, 2,4 - or 2,6-diaminotoluene, m- or p-xylylenediamine, isophoronediamine, bis(aminomethyl)bidicloheptane, 1,3- or 1,4-bis(aminomethyl)cyclohexane, 2- or 4-aminopiperidine, 2- or 4-aminomethylpiperidine, 2- or 4-aminoethylpiperidine, N-aminoethylmorpholine, and N-aminopropylmorpholine;
monofunctional secondary amine compounds such as diethylamine, dipropylamine, di-n-butylamine, di-sec-butylamine, diisobutylamine, di-n-pentylamine, di-3-pentylamine, dihexylamine, dioctylamine, di(2-ethylhexyl)amine, methyl hexylamine, diallylamine, N-methylallylamine, piperidine, pyrrolidine, diphenylamine, N-methylamine, N-ethylamine, dibenzylamine, N-methylbenzylamine, N-ethylbenzylamine, dicyclohexylamine, N-methylaniline, N-ethylaniline, dinaphthylamine, 1-methylpiperazine, and morpholine;
secondary polyamine compounds such as N,N'-dimethylethylenediamine, N,N'-dimethyl-1,2-diaminopropane, N,N'-dimethyl-1,3-diaminopropane, N,N'-dimethyl-1,2-diaminobutane, N,N'-dimethyl-1,3-diaminobutane, N,N'-dimethyl-1,4-diaminobutane, N,N'-dimethyl-1,5-diaminopentane, N,N'-dimethyl-1 ,6-diaminohexane, N,N'-dimethyl-1,7-diaminoheptane, N,N'-diethylethylenediamine, N,N'-diethyl-1,2-diaminopropane, N,N'-diethyl-1,3-diaminopropane, N,N'-diethyl-1,2-diaminobutane, N,N'-diethyl-1,3-diaminobutane, N,N'-diethyl-1,4-diaminobutane, N,N'-diethyl-1,5-diaminopentane, N,N'-diethyl-1,6-diaminohexane, N,N'-diethyl-1,7-diaminoheptane, piperazine, 2-methylpiperazine, 2,5-dimethylpiperazine, 2,6-dimethylpiperazine, homopiperazine, 1,1-di-(4-piperidyl)methane, 1,2-di-(4-piperidyl)ethane, 1,3-di-(4-piperidyl)propane, 1,4-di-(4-piperidyl)butane, and tetramethylguanidine; and the like.

The polymerizable composition may further include an episulfide compound having one or more episulfide groups.

Examples of the episulfide compound include an epithioethylthio compound, a chain aliphatic 2,3-epithiopropylthio compound, a cycloaliphatic 2,3-epithiopropylthio compound, and an aromatic 2,3-epithiopropylthio compound, a chain aliphatic 2,3-epithiopropyloxy compound, a cyclic aliphatic 2,3-epithiopropyloxy compound, and an aromatic 2,3-epithiopropyloxy compound and the like.

The episulfide compound may be used alone or in combination of two or more types.

Examples of the episulfide compound include episulfide compounds described in WO 2015/137401, WO 2017/159839, and JP 2018-154690, thioepoxy compounds described in JP 2002-194083, and novel tetrathiaspiro compounds described in JP-A No. 2019-1785.

The polymerizable composition may include an isocyanate compound (hereinafter also referred to as another isocyanate compound) other than the compound represented by the general formula (1) derived from a biomass raw material. Examples of another isocyanate compound include a compound represented by the general formula (1) that is not derived from a biomass raw material, pentamethylene diisocyanate, hexamethylene diisocyanate, m-xylylene diisocyanate, p-xylylene diisocyanate, isophorone diisocyanate, bis(isocyanatomethyl)cyclohexane, bis(isocyanatocyclohexyl)methane, 2,5-bis(isocyanatomethyl)bicyclo-[2.2.1]-heptane, 2,6-bis(isocyanatomethyl)bicyclo-[2.2.1]-heptane, tolylene diisocyanate, 4,4'-diphenylmethane diisocyanate, phenylene diisocyanate and the like.

Another isocyanate compound may be used alone or in combination of two or more. Another isocyanate compound (excluding compound represented by general the formula (1) that is not derived from biomass raw material) may or may not be derived from a biomass raw material.

In the polymerizable composition, the content of the compound represented by the general formula (1) derived from a biomass raw material may be from 50% by mass to 100% by mass, from 70% by mass to 100% by mass, or from 90% by mass to 100% by mass, with respect to the total amount of the isocyanate compound.

The mixing ratio of the isocyanate compound including the compound represented by the general formula (1) and the thiol compound, and the mixing ratio of the isocyanate compound or the thiol compound and the active hydrogen compound that may be included in the polymerizable composition as necessary are not particularly limited.

For example, the ratio of the charged mass of the thiol compound to the charged mass of the isocyanate compound (that is, charged mass [thiol compound/isocyanate compound]) is preferably from 0.10 to 10.0, more preferably from 0.20 to 5.00, further preferably from 0.50 to 1.50, and particularly preferably from 0.70 to 1.30.

For example, the molar ratio of the mercapto group included in the thiol compound and the isocyanato group of the isocyanate compound (mercapto group/isocyanato group) is preferably from 0.5 to 3.0, more preferably from 0.6 to 2.0, and further preferably from 0.8 to 1.3.

The total charged mass of the thiol compound and isocyanate compound is not particularly limited. For example, the total charged mass is preferably 60% by mass or more, more preferably 80% by mass or more, and further preferably 90% by mass or more, with respect to the total amount of the polymerizable composition.

The polymerizable composition may include a component (hereinafter also referred to as another component) other than the aforementioned compound. Examples of another component include a polymerization catalyst, an internal mold release agent, a resin modifier, a chain extender, a crosslinking agent, a radical scavenger, a light stabilizer, an ultraviolet absorber, an antioxidant, an oil-soluble dye, a filler, an adhesion improver, an antibacterial agent, an antistatic agent, a dye, an optical brightener, a fluorescent pigment, an inorganic pigment and the like.

Examples of the polymerization catalyst include a tertiary amine compound, an inorganic or organic acid salt thereof, a metal compound, a quaternary ammonium salt, an organic sulfonic acid, and the like.

As the internal mold release agent, an acidic phosphate ester, a silicone compound, or the like can be used. Examples of the acidic phosphoric acid ester include a phosphoric acid monoester and a phosphoric acid diester, each of which can be used alone or in combination of two or more types.

For example, ZelecUN manufactured by STEPAN, MR internal mold release agent manufactured by Mitsui Chemicals, Inc., JP series manufactured by Johoku Chemical Co., Ltd., Phosphanol (registered trademark) series manufactured by Toho Chemical Co., Ltd., and AP, or DP series manufactured by DAIHACHI CHEMICAL INDUSTRY CO., LTD. or the like can be used.

As the silicone compound, a polyether-modified silicone compound or the like can be used.

Examples of the resin modifier include an olefin compound including an episulfide compound, an alcohol compound, an amine compound, an epoxy compound, an organic acid, an organic acid anhydride, a (meth)acrylate compound, or the like. Herein, the (meth)acrylate compound means at least one of an acrylate compound or a methacrylate compound.

The above-mentioned components can be mixed according to a conventional method, and the mixing method is not particularly limited.

By curing the polymerizable composition, a cured product that includes the resin of the present disclosure can be obtained.

Curing of the polymerizable composition can be performed by polymerizing the monomers in the polymerizable composition (for example, isocyanate compound, thiol compound, and active hydrogen compound included as necessary). As a pretreatment for polymerization, the polymerizable composition may be subjected to treatments such as filtration and deaeration.

The method of curing the polymerizable composition may be either thermal curing or photocuring, or a combination of thermal curing and photocuring.

The polymerization conditions (for example, polymerization temperature, polymerization time, or the like) for polymerizing the monomers in the above polymerizable composition are set as appropriate in view of the composition of the polymerization composition, the type and amount of monomers in the composition, the type and amount used of polymerization catalyst in the composition, the properties of the mold when using a mold described later, or the like.

For example, when curing the polymerizable composition by thermosetting, examples of the polymerization temperature include from 0°C to 150°C, from 20°C to 130°C and the like.

Examples of the polymerization time include from 1 hour to 200 hours, from 1 hour to 80 hours, from 1 hour to 48 hours and the like.

The polymer obtained by polymerizing monomers may be subjected to a treatment such as annealing.

Examples of the annealing temperature include from 50°C to 150°C, from 90°C to 140°C, or from 100°C to 130°C.

The biomass degree of the resin of the present disclosure is preferably 35% or more, more preferably 50% or more. The biomass degree of the resin may be 85% or less, or 70% or less.

In the resin of the present disclosure, the refractive index ne is preferably 1.650 or more, more preferably 1.652 or more, and further preferably 1.654 or more.

In the resin of the present disclosure, the refractive index may be 1.750 or less.

The refractive index can be measured by the method described in the Examples below.

### <2,5-bis(isocyanatomethyl)furan>

2,5-bis(isocyanatomethyl)furan of the present disclosure is derived from 2,5-bis(aminomethyl)furan obtained from a biomass raw material. Examples of the biomass raw material include the aforementioned saccharide. A preferable form of 2,5-bis(isocyanatomethyl)furan of the present disclosure is the same as the preferable form of 2,5-bis(isocyanatomethyl)furan explained in the section of <Resin> described above.

### <Polymerizable composition>

The polymerizable composition of the present disclosure preferably include 2,5-bis(isocyanatomethyl)furan explained in the section of <Resin> described above or the 2,5-bis(isocyanatomethyl)furan derived from a biomass raw material of the present disclosure.

The polymerizable composition of the present disclosure may include a thiol compound having one or more mercapto groups or another active hydrogen compound, or may include aforementioned another component. The preferable form of the polymerizable composition is the same as the preferable form of the polymerizable composition explained in the section of <Resin> described above.

From the viewpoint of the refractive index of the resulting resin, the polymerizable composition of the present disclosure preferably includes 2,5-bis(isocyanatomethyl)furan explained in the section of <Resin> described above or 2,5-bis(isocyanatomethyl)furan derived from a biomass raw material of the present disclosure, and a thiol compound having one or more mercapto groups. Furthermore, the thiol compound more preferably includes at least one selected from the group consisting of 4-mercaptomethyl-1,8-dimercapto-3,6-dithiaoctane, 4,8-dimercaptomethyl-1,11-dimercapto-3,6,9-trithiaundecane, 4,7-dimercaptomethyl-1,11-dimercapto-3,6,9-trithiaundecane, and 5,7-dimercaptomethyl-1,11-dimercapto-3,6,9-trithiaundecane.

### <Molded body>

The molded body of the present disclosure includes the resin of the present disclosure described above.

The molded body of the present disclosure can be manufactured, for example, by cast polymerization.

In cast polymerization, first, the polymerizable composition is injected between a pair of molds held together by a gasket, tape, or the like. At this time, defoaming treatment, filtration treatment, or the like may be performed as necessary.

Next, by polymerizing the monomers in the polymerizable composition injected between the molds, the polymerizable composition is cured between the molds to obtain a cured product. Then, the cured product is removed from the molds to obtain the cured product. As a result, the molded body, which is a molded cured product, is obtained.

### <Optical material>

An optical material of the present disclosure includes the aforementioned molded body of the present disclosure.

The optical material of the present disclosure can be manufactured, for example, by the cast polymerization described above.

The optical material of the present disclosure may be made of the molded body of the present disclosure, or may include the molded body of the present disclosure and another element.

Examples of another element include another member, a coating layer provided on the molded body of the present disclosure, or the like.

Examples of the optical material of the present disclosure include a lens (for example, eyeglass lens, camera lens, polarized lens, or the like), a light emitting diode (LED), and the like.

### <Lens>

The lens of the present disclosure is an example of the optical material of the present disclosure, and includes the aforementioned molded body of the present disclosure.

The lens of the present disclosure can be manufactured, for example, by the cast polymerization described above.

Examples of the lens of the present disclosure include a spectacle lens, a camera lens, a polarized lens, and the like.

Hereinafter, a spectacle lens will be described as an example of the lens of the present disclosure.

The eyeglass lens includes the molded body of the present disclosure that is molded into a desired lens shape.

The spectacle lens preferably further includes a coating layer provided on one or both sides of the molded body.

Specific examples of the coating layer include a primer layer, a hard coat layer, an antireflection layer, an antifogging coat layer, an antifouling layer, a water repellent layer, and the like. These coating layers can be used alone or in a multilayered form of a plurality of coating layers.

When coating layers are applied to both sides of the molded body, the same coating layer or different coating layers may be applied to each side.

The component in the coating layer can be appropriately selected depending on the purpose.

Examples of the component in the coating layer include a resin (for example, urethane resin, epoxy resin, polyester resin, melamine resin, polyvinyl acetal resin, or the like), an infrared absorber, a light stabilizer, an antioxidant, a photochromic compound, a dye, a pigment an antistatic agent and the like.

Regarding the spectacle lens and coating layer, for example, descriptions in known documents such as JP 2002-194083 A and WO 2017/047745 can be appropriately referred to.

### EXAMPLES

Examples of the present disclosure will be shown below, but the present disclosure is not limited to the following examples.

Hereinafter, "room temperature" means 25°C unless otherwise specified.

First, evaluation methods in Examples of the present disclosure are shown below.

### <Evaluation methods>

### ▪Refractive index (ne), Abbe number (ve)

Using a Pulfrich refractometer KPR-30 manufactured by Shimadzu Corporation, a refractive index (ne, nF', nC') at wavelengths of 546.1 nm (mercury e-line), 480.0 nm (Cd F' line) and 643.9 nm (Cd C' line) were measured, and the refractive index (ne) and Abbe number (ve) of a molded body were determined.

### ▪Heat resistance

Using a thermomechanical analyzer TMA-60 manufactured by Shimadzu Corporation, the glass transition temperature Tg of a molded body was measured using the TMA penetration method (50g load, pin tip 0.5mmϕ, heating rate 10°C/min), and Tg was used as an index of heat resistance.

### ▪Specific gravity: it was measured by the Archimedes method.

### ▪Haze

Using a haze meter (NDH 2000 manufactured by Nippon Denshoku Kogyo Co., Ltd.), the haze value of a flat molded body (thickness: 2.5 mm) was measured.

Note that if the haze value is less than 0.5, it can be used as a lens without any problem.

### ▪pMC

### Test method

Using a ¹⁴C-AMS dedicated device (manufactured by NEC Corporation) based on a tandem accelerator, measurements of ¹⁴C counts, ¹³C concentration (¹³C/¹²C) and ¹⁴C concentration (¹⁴C/¹²C) were performed. In the measurement, oxalic acid (HOxII) provided by the National Institute of Standards and Technology (NIST) was used as a standard sample. Measurements of this standard sample and background sample were also performed at the same time.

### Calculation method

(1) δ¹³C is the value represented by measuring ¹³C concentration (¹³C/¹²C) of the sample carbon and expressed in 1,000 minutes deviation (‰) from the reference sample, and the value measured by a 14C-AMS device were used.
(2) pMC is the ratio of ¹⁴C concentration of the sample carbon to standard modern carbon. The value corrected by δ¹³C is shown.

### [Experimental Example 1]

### Synthesis of 2,5-bis(aminomethyl)furan hydrochloride

218.8 g of orthodichlorobenzene was added to 48.0 g of 2,5-bis(aminomethyl)furan and the mixture was stirred to prepare an orthodichlorobenzene solution including 18.0% by mass of 2,5-bis(aminomethyl)furan.

298.2 g of orthodichlorobenzene was added to a 1 L 5-necked flask, and after stirring, the internal temperature was raised to 93°C. After the temperature was raised to 93°C, hydrogen chloride gas was started to be blown into the orthodichlorobenzene at a rate of 0.27 g/min. Next, the prepared orthodichlorobenzene solution including 18.0% by mass of 2,5-bis(aminomethyl)furan was fed into the orthodichlorobenzene at a rate of 1.16 g/min to initiate the reaction. The internal temperature during the reaction was controlled from 93°C to 96°C.

After feeding the entire orthodichlorobenzene solution including 18.0% by mass of 2,5-bis(aminomethyl)furan over 3 hours and 50 minutes, hydrogen chloride gas was further blown in for 30 minutes.

Thereafter, the blowing of hydrogen chloride gas into the orthodichlorobenzene was stopped, nitrogen gas was blown into the orthodichlorobenzene, and the hydrogen chloride gas was degassed for 30 minutes. Finally, the reaction solution was returned to room temperature to obtain 617.3 g of an orthodichlorobenzene solution of 2,5-bis(aminomethyl)furan hydrochloride.

### Synthesis of 2,5-bis(isocyanatomethyl)furan

328.2 g of the orthodichlorobenzene solution of 2,5-bis(aminomethyl)furan hydrochloride prepared above and 41.7 g of orthodichlorobenzene were added to a 500 mL five-necked flask and the mixture was stirred, then the internal temperature was raised to 165°C. After the temperature was raised, phosgene gas was blown into the solution at a rate of 43.5 g/hour for 4 hours. The internal temperature during the reaction was controlled from 161°C to 168°C. After blowing in phosgene gas, the internal temperature was controlled from 155°C to 161°C, nitrogen gas was blown into the reaction solution, and phosgene gas was degassed for 2 hours.

The reaction solution was returned to room temperature to obtain 363.7 g of 2,5-bis(isocyanatomethyl)furan solution. The obtained 2,5-bis(isocyanatomethyl)furan solution was filtered and purified by distillation to obtain 17.8 g of 2,5-bis(isocyanatomethyl)furan.

### [Experiment example 2]

### Synthesis of 2,5-bis(isocyanatomethyl)furan

253.4 g of the orthodichlorobenzene solution of 2,5-bis(aminomethyl)furan hydrochloride prepared in Experimental Example 1 and 51.6 g of orthodichlorobenzene were added to a 500 mL five-necked flask, the mixture was stirred, and then the internal temperature was raised to 60°C. After the temperature was raised, phosgene gas was blown into the solution at a rate of 45.5 g/hour for 4 hours. The internal temperature during the reaction was made to reach 145°C 40 minutes after the start of phosgene gas blowing, and was controlled from 145°C to 150°C until 3 hours later. Thereafter, the internal temperature gradually decreased and reached 108°C after 4 hours. After blowing in phosgene gas, the internal temperature was controlled from 108°C to 150°C, nitrogen gas was blown into the reaction solution, and phosgene gas was degassed for 2 hours.

The reaction solution was returned to room temperature to obtain 300.1 g of 2,5-bis(isocyanatomethyl)furan solution. The obtained 2,5-bis(isocyanatomethyl)furan solution was filtered and purified by distillation to obtain 19.3 g of 2,5-bis(isocyanatomethyl)furan.

The ratio (pMC, With δ¹³C correction) of ¹⁴C concentration of carbon in the obtained 2,5-bis(isocyanatomethyl)furan was 62.99±0.22%, and the biomass degree was 63%.

### [Example 1]

0.01 part by mass of dibutyltin dichloride as a curing catalyst and 10 parts by mass of a polythiol composition mainly including 4,8-dimercaptomethyl-1,11-dimercapto-3,6,9-trithiaundecane, 4,7-dimercaptomethyl-1,11-dimercapto-3,6,9-trithiaundecane, and 5,7-dimercaptomethyl-1,11-dimercapto-3,6,9-trithiaundecane, which were synthesized according to the method described in International Publication No. 2021/010392 using epichlorohydrin derived from a biomass raw material, were mixed to prepare a catalyst master solution.

The ratio (pMC, With δ¹³C correction) of ¹⁴C concentration of carbon in the polythiol composition was 59.87±0.22%, and the biomass degree was 60%.

0.0052 parts by mass of an internal mold release agent for MR (manufactured by Mitsui Chemicals, Inc.) and 0.079 parts by mass of Biosorb 583 (manufactured by Kyodo Yakuhin Co., Ltd., 2-(2-hydroxy-5-tert-butyl-octylphenyl)benzotriazole) and 2.50 parts by mass of 2,5-bis(isocyanatomethyl)furan were mixed and dissolved to obtain a solution. 0.54 parts by mass of the catalyst master solution, and 2.24 parts by mass of a polythiol composition having the same biomass degree (biomass degree 60%) as above and mainly including 4,8-dimercaptomethyl-1,11-dimercapto-3,6,9-trithiaundecane, 4,7-dimercaptomethyl-1,11-dimercapto-3,6,9-trithiaundecane, and 5,7-dimercaptomethyl-1,11-dimercapto-3,6,9-trithiaundecane, which were synthesized according to the method described in International Publication No. 2021/010392 using epichlorohydrin derived from a biomass raw material, were added to the obtained solution, the components were mixed and dissolved to obtain a homogeneous solution. Next, after filtering through a 3 µm filter, defoaming was performed at 400 Pa for 30 minutes, and the uniform solution was poured into a mold. Thereafter, the mold into which the uniform solution was injected was put into a polymerization oven, and the temperature was gradually raised from 25°C to 120°C to polymerize and harden over 20 hours.

After the polymerization was completed, the cured product was taken out of the oven and released from the mold, and further annealed at 120°C for 2 hours to obtain a molded body.

The refractive index of the obtained molded body was 1.655 (e line), the Abbe number 33, Tg 88.4°C, the specific gravity 1.409, the ratio (pMC, With δ¹³C correction) of ¹⁴C concentration of carbon in the molded body was 60.07±0.19%, and the biomass degree of the resin constituting the molded body was 60%.

### [Example 2]

0.01 part by mass of dibutyltin dichloride as a curing catalyst and 10 parts by mass of a polythiol composition mainly including 4,8-dimercaptomethyl-1,11-dimercapto-3,6,9-trithiaundecane, 4,7-dimercaptomethyl-1,11-dimercapto-3,6,9-trithiaundecane, and 5,7-dimercaptomethyl-1,11-dimercapto-3,6,9-trithiaundecane, which were synthesized according to the method described in International Publication No. 2021/010392 using epichlorohydrin derived from a non-biomass raw material, were mixed to prepare a catalyst master solution.

The ratio (pMC, With δ¹³C correction) of ¹⁴C concentration of carbon in the polythiol composition was <0.13%, and the biomass degree was 0%.

0.0046 parts by mass of an internal mold release agent for MR (manufactured by Mitsui Chemicals, Inc.) and 0.069 parts by mass of Biosorb 583 (manufactured by Kyodo Yakuhin Co., Ltd., 2-(2-hydroxy-5-tert-butyl-octylphenyl)benzotriazole) and 2.20 parts by mass of 2,5-bis(isocyanatomethyl)furan were mixed and dissolved to obtain a solution. 0.46 parts by mass of the catalyst master solution, and 1.95 parts by mass of a polythiol composition having the same biomass degree (biomass degree 0% ) as above and mainly including 4,8-dimercaptomethyl-1,11-dimercapto-3,6,9-trithiaundecane, 4,7-dimercaptomethyl-1,11-dimercapto-3,6,9-trithiaundecane, and 5,7-dimercaptomethyl-1,11-dimercapto-3,6,9-trithiaundecane, which were synthesized according to the method described in International Publication No. 2021/010392 using epichlorohydrin derived from a non-biomass raw material, were added to the obtained solution, the components were mixed and dissolved to obtain a homogeneous solution. Next, after filtering through a 3 µm filter, defoaming was performed at 400 Pa for 30 minutes, and the uniform solution was poured into a mold. Thereafter, the mold into which the uniform solution was injected was put into a polymerization oven, and the temperature was gradually raised from 25°C to 120°C to polymerize and harden over 20 hours.

After the polymerization was completed, the cured product was taken out of the oven and released from the mold, and further annealed at 120°C for 2 hours to obtain a molded body.

The refractive index of the obtained molded body was 1.655 (eline), the Abbe number 32, Tg 88.9°C, the specific gravity 1.396, the ratio (pMC, With δ¹³C correction) of ¹⁴C concentration of carbon in the molded body was 36.29±0.16%, and the biomass degree of the resin constituting the molded body was 36%.

### [Example 3]

A molded body was obtained in the same manner as in Example 1, except that a mixture of a biomass raw material and a non-biomass raw material was used as epichlorohydrin.

The refractive index of the obtained molded body was 1.655 (e line), the Abbe number 32, Tg 85.8°C, the specific gravity 1.406, the ratio (pMC, With δ¹³C correction) of ¹⁴C concentration of carbon in the molded body was 40.19±0.16%, and the biomass degree of the resin constituting the molded body was 40%. Further, the haze of the molded body with a thickness of 2.5 mm was 0.34, and it had high transparency.

Furthermore, in Examples 1 to 3, the molded bodies with high transparency could be obtained without any special preparation method such as reacting the polymerizable compositions for 1 hour or more in advance.

On the other hand, in the comparative examples (for example, Comparative Examples B9b to B11b) described in the aforementioned Patent Document 1 (International Publication No. 2015/119220), from the viewpoint of preventing cloudiness of the lenses occurred in other comparative examples (for example, Comparative Examples B9 and B10), 90 minutes of reaction operation is required in advance. However, operations requiring a prior reaction may reduce the productivity of cured products such as lenses, and may make it difficult to inject the composition into a mold due to increased viscosity due to the reaction.

In the present disclosure, as shown in Examples 1 to 3, it is possible to produce a cured product such as a lens with high transparency without performing a reaction operation in advance, and the productivity of the cured product is excellent.

In particular, when the compound represented by the general formula (1) is 2,5-bis(isocyanatomethyl)furan represented by the formula (2), and the thiol compound includes at least one compound selected from the group consisting of 4-mercaptomethyl-1,8-dimercapto-3,6-dithiaoctane, 4,8-dimercaptomethyl-1,11-dimercapto-3,6,9-trithiaundecane, 4,7-dimercaptomethyl-1,11-dimercapto-3,6,9-trithiaundecane, and 5,7-dimercaptomethyl-1,11-dimercapto-3,6,9-trithiaundecane in the polymerizable composition, the composition tends to have excellent productivity of a cured product with excellent transparency. Furthermore, the cured product obtained using the polymerizable composition has a high biomass degree, a high refractive index, and high heat resistance.

The entire contents of the disclosures by Japanese Patent Application No. 2022-031204 filed on March 1, 2022, are incorporated herein by reference.

All the literature, patent application, and technical standards cited herein are also herein incorporated to the same extent as provided for specifically and severally with respect to an individual literature, patent application, and technical standard to the effect that the same should be so incorporated by reference.

## Claims

1. A resin, comprising a cured product of a polymerizable composition including a compound represented by the following general formula (1) and a thiol compound having one or more mercapto groups,
wherein a biomass degree of the resin is 15% or more: 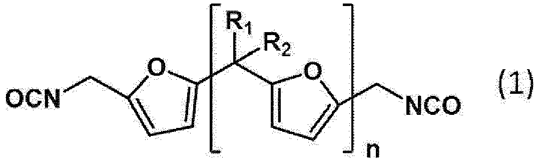
wherein, in the general formula (1), each of R₁ and R₂ independently represent a hydrogen atom or a methyl group, and n represents an integer of 0 or 1.

2. The resin according to claim 1, wherein the compound represented by the general formula (1) includes 2,5-bis(isocyanatomethyl)furan represented by the following formula (2):

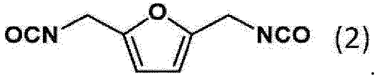

3. The resin according to claim 1 or 2, wherein the biomass degree of the resin is 35% or more.

4. The resin according to any one of claims 1 to 3, wherein the thiol compound includes at least one compound selected from the group consisting of 4-mercaptomethyl-1,8-dimercapto-3,6-dithiaoctane, 4,8-dimercaptomethyl-1,11-dimercapto-3,6,9-trithiaundecane, 4,7-dimercaptomethyl-1,11-dimercapto-3,6,9-trithiaundecane, and 5,7-dimercaptomethyl-1,11-dimercapto-3,6,9-trithiaundecane.

5. The resin according to any one of claims 1 to 4, wherein the polymerizable composition further includes an episulfide compound having one or more episulfide groups.

6. The resin according to any one of claims 1 to 5, wherein a refractive index ne of the resin is 1.650 or more.

7. A molded body, comprising the resin according to any one of claims 1 to 6.

8. An optical material, comprising the molded body according to claim 7.

9. A lens, comprising the molded body according to claim 7.

10. 2,5-bis(isocyanatomethyl)furan, having a biomass degree of 50% or more.

11. 2,5-bis(isocyanatomethyl)furan, derived from 2,5-bis(aminomethyl)furan obtained from a biomass raw material.

12. A polymerizable composition, comprising the 2,5-bis(isocyanatomethyl)furan according to claim 10 or 11.

13. The polymerizable composition according to claim 12, further comprising a thiol compound having one or more mercapto groups.

14. The polymerizable composition according to claim 12 or 13, further comprising an episulfide compound having one or more episulfide groups.
